# EUROPEAN PATENT APPLICATION

(11) **EP 4 748 852 A1**
(43) Date of publication of application: **27.05.2026**
(21) Application number: 24842054.9
(22) Date of filing: 22.05.2024
(51) Int. Cl.: C07K 14/47, C12N 15/70, C12N 15/12, C12N 1/21, A61K 38/17, A61P 25/00, A61P 31/12, A61P 25/08, A61P 11/14, A61P 11/10, A61P 29/00, A61P 9/12, C07K 1/34, C07K 1/30, C07K 1/16, C07K 1/18, C07K 1/36, C12R 1/19

(54) **KERATIN CF7, PREPARATION METHOD THEREFOR, PHARMACEUTICAL COMPOSITION CONTAINING SAME, AND USE OF KERATIN CF7**

(30) Priority: 17.07.2023 CN 202310875553
(71) Applicant: Institute of Materia Medica, Chinese Academy of Medical Sciences, Beijing 100050 (CN)
(72) Inventor: YU, Shishan, Beijing 100050 (CN); WANG, Xiaoliang, Beijing 100050 (CN); MA, Shuanggang, Beijing 100050 (CN); WANG, Xiaojing, Beijing 100050 (CN); QU, Jing, Beijing 100050 (CN); WANG, Ling, Beijing 100050 (CN); FENG, Nan, Beijing 100050 (CN); LI, Mi, Beijing 100050 (CN); XU, Shaofeng, Beijing 100050 (CN); CAI, Jie, Beijing 100050 (CN); LI, Yong, Beijing 100050 (CN); LIU, Yunbao, Beijing 100050 (CN); WANG, Weiping, Beijing 100050 (CN); ZHANG, Mi, Beijing 100050 (CN)
(74) Representative: Santarelli
(86) International application number: PCT/CN2024/094562
(87) International publication number: WO 2025/016054

(57) **Abstract**

The present invention relates to the technical field of medicines, provides a keratin CF7, a preparation method therefor, a pharmaceutical composition containing same, and a use of keratin CF7, and specifically provides keratin CF7, a nucleic acid molecule encoding keratin CF7, an expression vector containing the nucleic acid molecule, a host cell containing the expression vector or the nucleic acid molecule integrated into a genome, a preparation method for keratin CF7, a pharmaceutical composition containing keratin CF7, and a use of keratin CF7, the nucleic acid molecule, the expression vector, the host cell, or the pharmaceutical composition in preparation of drugs having antipyretic and analgesic, antitussive and expectorant, anticonvulsant, antiepileptic, antihypertensive, anti-inflammatory and antiviral effects.

## Description

### Technical field

The present invention pertains to the field of medical technology, and relates to a keratin CF7, a nucleic acid molecule encoding the keratin CF7, an expression vector containing the nucleic acid molecule, and a host cell containing the expression vector or genomically integrated with the nucleic acid molecule, a method for preparing the keratin CF7, a pharmaceutical composition containing the keratin CF7, and use of the keratin CF7 and the pharmaceutical composition in the preparation of a medicament for antipyretic and analgesic, antitussive and expectorant, anticonvulsant, antiepileptic, hypotensive, anti-inflammatory, or antiviral therapy.

### Background Art

Keratin is a kind of protein, which is widely found in the epidermis of humans and animals, and is the main component of hair, feathers, hoofs, shells, claws, horns, etc. It is an extremely important structural protein for connective tissue and plays a role in protecting the body.

Keratin is widely present in organisms and is a renewable resource with great utilization value, but it has not been widely and effectively used. The main reason is that keratin is insoluble in various solvents, and keratin is generally more resistant to enzymatic hydrolysis by proteases than other proteins. Therefore, it is very difficult to extract and prepare natural keratin.

With the rapid development of modern biotechnology such as genomics, proteomics, genetic engineering, and microbial engineering, more and more genes have been discovered. The use of protein expression systems to prepare and produce target proteins is an important method for studying the biological functions of genes or proteins.

It is novel and innovative and is not reported in other literatures to prepare the target keratin by utilizing the protein expression system, and further to study its structure and function.

### Summary of the invention

The technical problem solved by the present invention is to provide a keratin CF7, a nucleic acid molecule encoding the keratin CF7, an expression vector containing the nucleic acid molecule, and a host cell containing the expression vector or genomically integrated with the nucleic acid molecule, a method for preparing the keratin CF7, a pharmaceutical composition containing the keratin CF7, and use of the keratin CF7, nucleic acid molecule, expression vector, host cell, or pharmaceutical composition in the preparation of a medicament for antipyretic and analgesic, antitussive and expectorant, anticonvulsant, antiepileptic, hypotensive, anti-inflammatory, or antiviral therapy.

To solve the technical problems of the present invention, the present invention provides the following technical solutions:
The first aspect of the technical solution of the present invention is to provide a keratin CF7, wherein the amino acid sequence of the keratin CF7 is as follows:
(1) the amino acid sequence shown in SEQ ID NO. 1 in the sequence listing; or
(2) an amino acid sequence obtained by substituting, deleting, or adding 1 to 35 amino acids in the amino acid sequence shown in SEQ ID NO. 1 in the sequence listing, and the keratin CF7 substantially maintains the same biological function.

Further, conventional modifications can be performed on keratin CF7; or a tag for detection or purification can be also attached to keratin CF7.

Furthermore, the conventional modification is acetylation, amidation, cyclization, glycosylation, phosphorylation, alkylation, biotinylation, fluorophore modification, polyethylene glycol PEG modification, immobilization modification, sulfation, oxidation, methylation, deamidation, disulfide bond formation, or disulfide bond cleavage; or the tag is His6, GST, EGFP, MBP, Nus, HA, IgG, FLAG, c-Myc, or Profinity eXact.

The second aspect of the technical solution of the present invention provides a nucleic acid molecule encoding the keratin CF7 of the first aspect.

Further, the nucleotide sequence of the nucleic acid molecule is:
(1) the nucleotide sequence shown in SEQ ID NO. 2 in the sequence listing;
(2) a nucleotide sequence obtained by sequence optimization based on the nucleotide sequence shown in SEQ ID NO. 2; or
(3) a nucleotide sequence complementary to the nucleotide sequence in (1) or (2) above.

The third aspect of the technical solution of the present invention provides an expression vector, wherein the expression vector comprises the nucleic acid molecule described in the second aspect.

Further, the expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K, etc.; the preferred expression vector is the pET series vector; the most preferred expression vector is pET-28a(+).

The fourth aspect of the technical solution of the present invention provides a host cell, wherein the host cell comprises the expression vector of the third aspect, or the nucleic acid molecule of the second aspect is integrated into the genome of the host cell.

Further, the host cell is a bacteria, a yeast, an aspergillus, a plant cell, or an insect cell.

Furthermore, the bacteria is *Escherichia coli* or yeast.

Competent host cells can be BL21 series, Transetta series, Rosetta series, DH5α series, JM series, Top series, Orgami series, Trans1-T1, TG1, TB1, Y11430, MG1003, GS115 (AOX1), KM71, SMD1168, etc.. The preferred expression competent cell is BL21 (DE3) or Transetta (DE3).

The fifth aspect of the technical solution of the present invention provides a method for preparing the keratin CF7 of the first aspect, comprising the following steps:
A. the nucleic acid molecule corresponding to the keratin CF7 of the first aspect is synthesized, the nucleic acid molecule is ligated into a corresponding expression vector, the expression vector is transformed into a host cell, the host cell carrying the expression vector is cultured under a certain condition in a fermentation device, and the expression of keratin CF7 is induced, to obtain a crude protein solution containing keratin CF7; and
B. the crude protein solution expressed in step A is separated, purified, and dried to obtain keratin CF7.

Further, in step A, the host cell is primarily Escherichia coli, the keratin CF7 is expressed in Escherichia coli inclusion bodies, and the fermentation device is a shake flask or a fermenter.

Further, in step A, after inducing the expression of keratin CF7, impurities can be washed with a washing agent and then dissolved with a urea solution to obtain a crude protein solution.

Further, the medium in step A can be LB medium, TB medium, SB medium, SOB medium, SOC medium, PDA medium, YPD medium, Bengal rose culture medium, high salt Czapek medium, DOBA medium, rice koji medium and its modified formula, etc.; LB medium or TB medium is preferred for shake flask fermentation, and TB medium is most preferred; LB medium and and its modified formula is preferred for fermentation tank.

Further, the inducer in step A can be IPTG, lactose, arabinose, etc.; preferred is IPTG or lactose.

Further, in step A, the obtained fermentation broth is centrifuged to discard the supernatant. The precipitate is suspended in a buffer, followed by bacterial cell disruption and subsequent centrifugation to remove the supernatant. After the precipitate is washed with a detergent, it is then dissolved in a urea solution to obtain a CF7 crude protein solution.

Among them, the buffer is preferably buffer A, and its dosage is: fermentation broth volume: buffer A volume = 1~100:1, preferably 10: 1.

The cleaning agent can be urea solution, guanidine hydrochloride solution, Triton and buffer A, etc., preferably urea solution, most preferably 2M urea solution (may contain 0.5% Triton) and 3M urea solution. The dosage is: fermentation liquid volume: urea solution volume = 0.2~100:1, preferably 1~15: 1.

The urea solution is preferably a 4M~8M urea solution, most preferably 8M urea solution and its dosage is: fermentation liquid volume:8M urea volume = 0.2~100: 1, preferably 2~15: 1.

Further, in step B, the method of separation and purification is ultrafiltration and microfiltration membrane technology purification method, column chromatography purification method, salting-out method, or dialysis method.

Further, in step B, the method of separation and purification is as follows:
(1) The dialysis method is to purify the crude protein solution obtained in step A by the dialysis method to obtain the target protein CF7 solution.
   The molecular weight cut-off of the dialysis bag can be 0.5-10 kD, the preferred molecular weight cut-off of the dialysis bag is 3.5-10kD, and the most preferred molecular weight cut-off of the dialysis bag is 10kD.
(2) The method for ultrafiltration and microfiltration is to purify the crude protein solution obtained in step A by membrane technology such as ultrafiltration membrane or microfiltration membrane to obtain a concentrated solution of the target protein CF7.
(3) The column chromatography method is to pass the crude protein solution obtained in step A through column chromatography, such as various exchange column or exclusion column chromatography, to separate and purify, to obtain the target protein CF7.

The preferred exclusion column is dextran gel column, Superdex 30 Increase, Superdex 75 Increase, Superdex 200 Increase, and Superose 6 Increase, etc.. The preferred exchange column is ion exchange resin column: anion exchange resin column, HiTrap Q FF, HiTrap Capto Q ImpRes, Capto Q ImpRes, HiTrap Capto Q, HiTrap DEAE, Toyopearl Q-650M and Toyopearl SuperQ-650M, etc.; cation exchange resin column, HiTrap SP FF, HiTrap Capto SP ImpRes, Capto SP ImpRes, HiTrap Capto SP, Toyopearl SP-650M, or Toyopearl Super SP-650M.

The eluent can be any eluent commonly used in the art, such as water, saline solution. The saline solution is sodium chloride solution, sodium dihydrogen phosphate solution, disodium hydrogen phosphate solution, sodium acetate, acetic acid, and the like.

(4)The salting-out method is to purify the crude protein solution obtained in step A by salting-out method to obtain the target protein CF7 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

Further, the target protein CF7 solution purified in step B can be freeze-dried or vacuum-dried into dry powder, or the concentrated solution can be directly spray-dried into dry powder.

The sixth aspect of the technical solution of the present invention provides a pharmaceutical composition, wherein the pharmaceutical composition comprises the keratin CF7 described in the first aspect or the nucleic acid molecule described in the second aspect or the expression vector in the third aspect or the host cell in the fourth aspect and a pharmaceutically acceptable carrier or excipient.

The keratin obtained in the above steps of the present invention can be freeze-dried or vacuum-dried into dry powder, or the concentrated liquid can be directly spray-dried into dry powder, and then made into various dosage forms.

The present invention relates to a pharmaceutical composition, which comprises any keratin obtained in the above steps and a pharmaceutically acceptable carrier.

The present invention also relates to a pharmaceutical composition containing the keratin of the present invention as an active ingredient and conventional a pharmaceutical excipient or adjuvant. Generally, the keratin of the present invention accounts for 0.1-100.0% of the total weight of the pharmaceutical composition.

The present invention also provides a pharmaceutical composition, which comprises a pharmaceutical effective dose of protein as an active ingredient and a pharmaceutically acceptable carrier.

The pharmaceutical composition of the present invention can be prepared according to methods recognized in the field. When used for this purpose, if necessary, the protein of the present invention can be combined with one or more solid or liquid pharmaceutical excipients and/or adjuvants to prepare an appropriate administration form or dosage form that can be used as human drugs or veterinary drugs.

The keratin of the present invention or the pharmaceutical composition containing the same can be administered in a unit dosage form. The route of administration can be enteral or parenteral, such as oral, intramuscular, subcutaneous, nasal, oral mucosa, ocular, pulmonary, transdermal, vaginal, peritoneal and rectal, etc., oral administration is preferred.

The keratin protein of the present invention or the pharmaceutical composition comprising the same can be administered by injection. Injections include intravenous injection, intramuscular injection, subcutaneous injection, intradermal injection, intraperitoneal injection, and acupoint injection, etc.

The dosage form for administration can be a liquid dosage form, a solid dosage form or a semi-solid dosage form. Liquid dosage forms can be solutions (including true solutions and colloidal solutions), emulsions (including oil-in-water, water-in-oil and double emulsions), suspensions, injections (including water injections, powder injections and infusions), eye drops, nasal drops, lotion and liniment, etc. The solid dosage form can be tablets (including ordinary tablets, enteric-coated tablets, buccal tablets, dispersible tablets, chewable tablets, effervescent tablets, orally disintegrating tablets), capsules (including hard capsules, soft capsules, and enteric-coated capsules), granules, powders, pellets, dripping pills, suppositories, films, patches, air (powder) sprays, sprays, etc.; semi-solid dosage forms can be ointments, gels, pastes, etc.

The keratin of the present invention can be made into ordinary preparations, slow-release preparations, controlled-release preparations, targeted preparations, and various particle delivery systems.

In order to make a unit administration dosage form into a tablet, various excipients known in the art can be widely used, including diluents, binders, wetting agents, disintegrants, lubricants, glidants. The diluent can be starch, dextrin, sucrose, glucose, lactose, mannitol, sorbitol, xylitol, microcrystalline cellulose, calcium sulfate, calcium hydrogen phosphate, calcium carbonate, etc.; the wetting agents can be water, ethanol, isopropanol, etc.; the binder can be starch slurry, dextrin, syrup, honey, glucose solution, microcrystalline cellulose, acacia slurry, gelatin slurry, sodium carboxymethyl cellulose, methyl cellulose, hydroxypropyl methyl cellulose, ethyl cellulose, acrylic resin, carbomer, polyvinylpyrrolidone, polyethylene dipropyl alcohol, etc.; the disintegrant can be dry starch, microcrystalline cellulose, low-substituted hydroxypropyl cellulose, cross-linked polyvinylpyrrolidone, cross-linked sodium carboxymethyl cellulose, sodium carboxymethyl starch, sodium bicarbonate and citric acid, calcium carbonate, polyoxyethylene sorbitol fatty acid ester, dodecyl sodium sulfonate; the lubricant and glidant can be talc, silicon dioxide, stearate, tartaric acid, liquid paraffin, polyethylene glycol, etc.

The tablets can also be further made into coated tablets, such as sugar-coated tablets, film-coated tablets, enteric-coated tablets, or double-layer tablets and multilayer tablets.

In order to make the administration unit into a pill, various carriers known in the field can be widely used. Examples of carriers are, for example, diluents and absorbents, such as glucose, lactose, starch, cocoa butter, hydrogenated vegetable oil, polyvinylpyrrolidone, polyethylene glycol laurate, kaolin, talc, etc.; binders, such as Gum arabic, xanthan gum, gelatin, ethanol, honey, liquid sugar, rice paste or batter, etc.; disintegrants, such as agar powder, dried starch, alginate, sodium lauryl sulfonate, methyl cellulose, ethyl cellulose, etc..

In order to make the administration unit into a suppository, various carriers known in the field can be widely used. Examples of carriers are, for example, polyethylene glycol, lecithin, cocoa butter, higher alcohols, higher alcohol esters, gelatin, semisynthetic glycerides and the like.

In order to make the administration unit into a capsule, the keratin of the present invention as an active ingredient is mixed with the above-mentioned various carriers, and the resulting mixture is placed into hard gelatin capsules or soft capsules. The keratin of the present invention as an active ingredient can also be made into microcapsules, suspended in an aqueous medium to form a suspension, or filled into hard capsules or made into injections for application.

For example, the keratin of the present invention is prepared into injection preparations, such as solutions, suspension solutions, emulsions and freeze-dried powder injections. Such preparations can be aqueous or non-aqueous and may contain one and/or more pharmacodynamically acceptable carriers, diluents, binders, lubricants, preservatives, surfactants or dispersants. For example, the diluent can be selected from water, ethanol, polyethylene glycol, 1,3-propylene glycol, ethoxylated isostearyl alcohol, polyoxylated isostearyl alcohol, polyoxyethylene sorbitol fatty acid esters and the like. In addition, in order to prepare an isotonic injection, an appropriate amount of sodium chloride, glucose or glycerin can be added to the injection preparation. In addition, conventional auxiliary solvents, buffers, pH adjusters, etc. can also be added. These auxiliary materials are commonly used in this field.

In addition, if necessary, coloring agents, preservatives, flavors, corrigents, sweeteners or other materials can also be added to the pharmaceutical preparations.

In order to achieve the purpose of medication and enhance the therapeutic effect, the keratin or the pharmaceutical composition of the present invention can be administered by any known administration method.

The administration dosage of the keratin pharmaceutical composition of the present invention depends on many factors, such as the nature and severity of the disease to be prevented or treated, the gender, age, weight, personality and individual response of the patient or animal, the route of administration, the frequency of administrations and the purpose of treatment, therefore the therapeutic dose of the present invention can have a wide range of changes. Generally speaking, the dosage of the pharmaceutical ingredients of the present invention is well known to those skilled in the art. Appropriate adjustments can be made according to the actual amount of the drug contained in the final preparation of the keratin composition of the present invention to meet the requirement of the therapeutically effective amount and accomplish the preventive or therapeutic purpose of the present invention. The appropriate daily dosage range of the keratin of the present invention: the dosage of the keratin of the present invention is from 0.01 to 500 mg/kg body weight, preferably from 0.5 to 100 mg/kg body weight, more preferably from 1 to 50 mg/kg body weight, and most preferably from 2 to 30 mg/kg body weight. The above dosage can be administered in a single dosage form or divided into several, such as two, three or four dosage forms for administration, depending on the clinical experience of the administering doctor and the dosage regimens including the use of other treatments. The total dose required for each treatment can be divided into multiple or single doses for administration. The protein or pharmaceutical composition of the present invention can be taken alone or be used in combination with other therapeutic drugs or symptomatic drugs with adjusting the dose.

The seventh aspect of the technical solution of the present invention provides the use of the keratin CF7 in the first aspect or the nucleic acid molecule in the second aspect or the expression vector in the third aspect or the host cell in the fourth aspect or the pharmaceutical composition in the sixth aspect in the preparation of a medicament for the antipyretic, analgesic, antitussive, expectorant, anticonvulsant, antiepileptic, hypotensive, anti-inflammatory, or antiviral therapy.

To accomplish the purpose of the present invention, the present invention takes the following technical solutions. Specifically, the preparation of keratin CF7 of the present invention includes the following steps:
(1) the nucleotide sequence is synthesized and the accuracy of the sequence is determined. The preferred nucleotide sequence is shown in SEQ ID No. 2.
(2) the nucleotide sequence is transfered into an expression vector.

The expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K etc. The preferred expression vector is the pET series vector. The most preferred expression vector is pET-28a(+).

(3) the expression vector is transfected into a host cell.

The host cell can be Escherichia coli or yeast. The preferred host cell is Escherichia coli.

The competent cells can be BL21 series, Transetta series, Rosetta series, DH5α series, JM series, Top series, Orgami series, Trans1-T1, TG1, TB1; Y11430, MG1003, GS115 (AOX1), KM71, SMD1168, etc. The preferred competent cell for expression is BL21(DE3) or transetta (DE3).

(4) The host cell is performed the fermentation culture to induce the expression of the target protein CF7 under appropriate conditions.

Fermentation equipment can use shake flasks or fermentation tanks.

The medium can be LB medium, TB medium, SB medium, SOB medium, SOC medium, PDA medium, YPD medium, Bengal rose medium, high salt Czapek medium, DOBA medium, rice koji culture medium and its modified formula, etc.. LB medium or TB medium is preferred for shake flask fermentation, and TB medium is most preferred. LB medium and its modified formula is preferred for fermentation tank.

The inducer can be IPTG, lactose, arabinose, etc.. The preferred is IPTG or lactose.

(5) The target protein CF7 is enriched.

The fermentation broth obtained in step (4) is centrifuged, and the supernatant is discarded. The precipitate is suspended in the buffer, the bacterial cell is broken, centrifuged again, and the supernatant is discarded. After the precipitation is washed with detergents, it is dissolved in a urea solution to obtain a CF7 crude protein solution.

Among them, the buffer is preferably buffer A, and its dosage is: fermentation broth volume: buffer A volume = 1~100: 1, preferably 5~10: 1;

The cleaning agent can be urea solution, guanidine hydrochloride solution, Triton, buffer A, etc., preferably urea solution, most preferably 2M urea solution (may contain 0.5% Triton). The dosage is: fermentation broth volume: urea solution volume = 0.2~100: 1, preferably 1~15: 1. The washing times is 3~10 times, preferably 6 times.

The urea solution is preferably a 4M~8M urea solution, most preferably 8M urea solution. Its dosage is: fermentation broth volume: 8M urea solution volume= 0.2~100:1, preferably 2~15: 1.

(6) The target protein CF7 is separated and purified.

The crude protein solution obtained in step (5) needs to be purified to obtain the target protein CF7. The purification can be carried out by dialysis, ultrafiltration and microfiltration, column chromatography, or salting out steps.
A. The dialysis step is to purify the crude protein solution obtained in step (5) by a dialysis method to obtain the target protein CF7 solution.

The molecular weight cut-off of the dialysis bag can be 0.5-10 kD, the preferred molecular weight cut-off of the dialysis bag is 3.5-10 kD, and the most preferred molecular weight cut-off of the dialysis bag is 10 kD.

B. The step of ultrafiltration and microfiltration is to purify the crude protein solution obtained in step (5) by membrane technology such as ultrafiltration membrane or microfiltration membrane to obtain the target protein CF7 concentrated solution.

C. The column chromatography step is to pass the crude protein solution obtained in step (5) through column chromatography, such as various exchange column or exclusion column chromatography, to separate and purify and to obtain the target protein CF7.

The preferred exclusion column is dextran gel column, Superdex 30 Increase, Superdex 75 Increase, Superdex 200 Increase, Superose 6 Increase, etc.. The preferred exchange column is ion exchange resin column: anion exchange resin column, HiTrap Q FF, HiTrap Capto Q ImpRes, Capto Q ImpRes, HiTrap Capto Q, HiTrap DEAE, Toyopearl Q-650M, Toyopearl SuperQ-650M, etc.; Cation exchange resin column, HiTrap SP FF, HiTrap Capto SP ImpRes, Capto SP ImpRes, HiTrap Capto SP, Toyopearl SP-650M, or Toyopearl Super SP-650M. The most preferred is anion exchange resin column.

The eluent can be any eluent commonly used in the art, such as water, saline solution. The saline solution includes sodium chloride solution, sodium dihydrogen phosphate solution, disodium hydrogen phosphate solution, sodium acetate, acetic acid, and the like.

D. The salting-out step is to purify the crude protein solution obtained in step (5) by a salting-out method to obtain the target protein CF7 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

The target protein CF7 solution purified from steps A to D can be freeze-dried or vacuum-dried into dry powder, or the concentrated solution can be directly spray-dried into dry powder.

The beneficial technical effects of the present invention:
1. The protein of the present invention is a keratin obtained for the first time, and the preparation method of the present invention has the characteristics of high yield and high sample purity.
2. The present invention studies the efficacy of the protein CF7 on the yeast-induced SD rat fever model, proving that the protein CF7 (50 mg/kg) has a significant effect in reducing the elevated body temperature of the model animals at 6 hours post-modeling.
3. The present invention studies the efficacy of the protein CF7 on the antitussive effect by the ammonia water-induced cough method in mice, demonstrating that the protein CF7 can reduce the number of coughs and possesses an antitussive effect.
4. The present invention studies the efficacy of the protein CF7 on expectoration by phenol red excretion method in mice, proving that the protein CF7 has a significant expectorant effect.
5. The present invention studies the efficacy of the protein CF7 on acetic acid writhing in ICR mice, proving that the protein CF7 can significantly reduce the number of writhing times in mice and has a significant analgesic effect.

### Figure Description

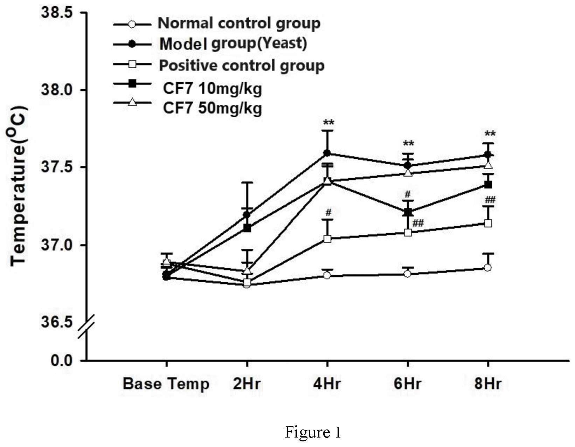

Figure 1: Effect of protein CF7 on the yeast-induced fever model in rats.

(Compared with the normal control group, ** P<0.01. Compared with the model group, # P<0.05, ## P<0.01).

### Examples

The following examples and pharmacological activity test examples are used to further illustrate the present invention, but this does not mean any limitation to the present invention.

The experimental methods in the following examples and pharmacological activity test examples are conventional methods unless otherwise specified; the experimental materials used, unless otherwise specified, are purchased from conventional biochemical reagent companies.

### Example 1 : Preparation of protein CF7 crude solution A by shake flask fermentation (TB medium)

The nucleotide sequence shown in SEQ ID No. 2 was synthesized and transferred into a pET-28a(+) vector. It was confirmed by sequencing that an expression vector comprising the correct sequence was obtained. And the expression vector was transfected into BL21 (DE3) cells and expression competent host cells comprising the target nucleotide sequence were obtained. The expression competent host cells were added into LB medium and cultured in a shaker at 37°C and 220 rpm for 1 hour to obtain a recombinant strain.

The recombinant strain was dipped and streaked in LBA plates containing Kanamycin. The plates were placed upside down in a 37°C constant temperature incubator and incubated overnight for 16 hours.

400 mL of TB medium was prepared, divided into 2 bottles, each bottle of 200 mL. Kanamycin (final concentration 50 µg/mL) was added to each bottle (200 mL) of TB medium. A single colony on the plate was taken and added to the TB medium and amplified and cultured overnight at 37°C and 220 rpm in the shaker to obtain seed liquid.

28.8L of TB medium was prepared and divided into 144 bottles, 200 mL per bottle. Kanamycin (final concentration 50 µg/mL) was added to each bottle (200 mL) of TB medium, then 2 mL of seed solution was added, and cultured in a shaker at 37°C and 220 rpm for 2-3 hours. The OD600 was monitored, when the OD600 reached about 1.0, an inducer was added to induce protein expression in a shaker, and the induction conditions were selected from the following table.

| | Inducer | Induction temperature | Induction time | Shaker speed |
|---|---|---|---|---|
| Inducing conditions | IPTG (Final concentration 0.5 mM) | 16 °C | 16 h | |
| | | 25 °C | 8 h | 220 rpm |
| | | 37 °C | 5 h | |

Each bottle of bacterial solution was combined, centrifuged at 7000 rpm for 5 minutes, and the supernatant was discarded after sterilization. The precipitate was suspended in about 3 L of buffer, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes, the supernatant was discarded, and the precipitate (i.e. inclusion body) was obtained. The precipitate was washed with detergent (three times with 1 L of 2M urea-0.5% Triton solution, two times with 1 L of 3M urea solution), centrifuged, and the supernatant was discarded. The precipitate was then dissolved in 1 L of 8M urea solution, and a crude protein solution A was obtained.

### Example 2 : Preparation of protein CF7 crude solution B by shake flask fermentation (other culture medium)

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained.

20 mL of LB medium was prepared, and 50µL of host cells comprising the target nucleotide sequence were added into 800µL of LB medium and were cultured in a shaker at 37°C and 220 rpm for 1 hour.

The above bacterial solution was dipped and streaked on an LBA plate containing kanamycin, and the plate was inverted and placed in a 37°C constant temperature incubator for overnight culture for 16 hours.

10 mL of LB medium was obtained and added with Kanamycin (final concentration 50µg/mL). Single colonies were taken, added into LB medium and were cultured overnight at 37°C and 220 rpm for 15 hours, then the seed solution was obtained.

1L of medium as indicated in the table below was prepared and divided into 10 bottles, 100 mL each. Kanamycin (final concentration 50µg/mL) was added to each bottle (100 mL) of medium, and 1 mL of seed solution was added and cultured for 2-3 hours at 37 °C and 220 rpm in a shaker. OD600 was monitored, and when OD600 reached about 1.0, the inducer IPTG (final concentration 0.5 mM) was added, and the protein expression was induced in a shaker at 37 °C and 220 rpm.

| | |
|---|---|
| Culture medium | LB medium, SOB medium, SOC medium |

Each bottle of bacterial liquid was combined and was centrifuged at 10000 rpm for 10 minutes, and the supernatant was discarded after sterilization. The precipitate was suspended into about 100 mL of buffer, filtered with an 80-100 mesh screen, and the filtrate was crushed with a high-pressure crusher at a pressure of 800-1000 bar, twice, 2 minutes each time. The broken bacteria liquid was centrifuged at 10000 rpm for 30 minutes and the supernatant was discarded.

The precipitate was firstly added with 40 mL of detergent buffer A, washed and centrifuged, and the supernatant was discarded. Then, the precipitate was added with 40 mL of wash solution containing 2M urea solution (with 0.5% Triton), washed twice and centrifuged, and the supernatant was discarded. Subsequently, the precipitate was added with 40 mL of 2M urea solution, washed twice and centrifuged, and the supernatant was discarded after each wash. Finally, the precipitate was added with 40 mL of 8M urea solution, and a crude protein solution B was obtained.

### Example 3 : Preparation of protein CF7 crude solution C in a fermentation tank

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained. The expression competent host cells were added into LB medium and cultured in a shaker at 37°C and 220 rpm for 1 hour to obtain a recombinant strain.

100µL of the recombinant strain was added to an LBA plate containing Kanamycin, spread evenly with a spreader until dry, and the plate was placed upside down in a constant temperature incubator at 37°C for overnight culture. Three single colonies were taken separately, streaked on plates containing Kanamycin, and then the plates were cultured overnight. After the verifications of three batches of shake flask fermentation and expression were confirmed to be correct, the strains were preserved with 15% glycerol and were divided into 0.8 mL for each to obtain a working cell bank, which was stored in a refrigerator at -80°C for later use.

100µL of 1 glycerol bacteria stock was taken out from the working cell bank, added with 40 mL of LB medium, added with Kanamycin (final concentration 50µg/mL), cultured in a shaker at 37°C and 220 rpm for 6 hours to obtain a first-level seed liquid.

1.2mL of the first-level seed liquid was taken, added to 120 mL of LB medium, added with Kanamycin (final concentration 50µg/mL), and then cultured in a shaker at 37°C and 220 rpm for 7 hours to obtain a second-level seed liquid.

3L of modified LB broth was added to a 5L fermentation tank, then added with 120 mL of the second-level seed liquid and 3 mL of Kanamycin (final concentration 50 µg/mL) and cultured at 37°C and 30% dissolved oxygen (series speed) for about 8 hours. The OD value was monitored to be about 20, and 3g lactose was used as an inducer. Induction was performed at 20°C, fed at a rate of 30mL/hour, and cultured at 20°C for 24 hours.

The bacteria liquid was centrifuged at 7000 rpm for 5 minutes and the supernatant was discarded after sterilization. The precipitate was suspended in about 600 mL of buffer A, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes and the supernatant was discarded.

The precipitate was washed 3 times with 2M urea solution (containing 0.5% Triton), 1L each time. Then the precipitate was added with 1L of 2M urea solution, washed twice and centrifuged, and the supernatant was discarded. Subsequently, the precipitate was added with 1L of 8M urea solution, washed one time and centrifuged, and the supernatant was discarded. Finally, the precipitate was added with 2L of 8M urea solution, and a crude protein solution C was obtained.

### Example 4 : Preparation of protein CF7 from protein crude solution A by dialysis

The protein crude solution A obtained in Example 1 was filtered with a 0.45 µm filter membrane, and the filtrates were combined. The filtrate was dialyzed with water, and the molecular weight cutoff of the dialysis bag was 10 kD. After 72 hours of dialysis, the inner liquid was freeze-dried to obtain the target protein CF7.

### Confirmation of the structure of protein CF7 by LC-MS/MS-based complete protein sequence analysis

Main materials: acetonitrile, formic acid, ammonium bicarbonate, dithiothreitol (DTT), iodoacetamide (IAA), trypsin, chymotrypsin, Glu-C, Asp-N;

Main instruments: Capillary High Performance Liquid Chromatograph (Thermo Ultimate 3000), Electrospray coupled Ion Trap Orbitrap Mass Spectrometer (Thermo Q Exative Hybrid Quadrupole-Orbitrap Mass Spectrometer).

### Methods and results:

Protein CF7 was pre-treated including dissolution replacement, reductive alkylation, and multiple proteolysis to obtain enzyme-cleaved peptide fragments. The solution of enzyme-cleaved peptides was analyzed by liquid chromatography tandem mass spectrometry. The original mass spectrometry file was retrieved from the protein database using Maxquant (1.6.2.10) for analysis data. The identification results determined that it was consistent with the target sequence of SEQ ID No.1.

### Example 5 : Preparation of protein CF7 by purification from protein crude solution A through salting-out method

The protein crude solution A was placed in a stirred container for salting out twice. Saturated ammonium sulfate solution was slowly added along the wall to make the final concentration of ammonium sulfate reach 25% or 50%. During the salting-out process, the protein was separated out. After the salting-out was completed, the solution was filtered, and the first salting out was finished. Then 400 mL of pure water was added into the precipitate to suspend, and a saturated solution of ammonium sulfate was slowly added along the wall again to make the final concentration of ammonium sulfate reach 25%. The second salting out and filtration were carried out, and the precipitate was the crude protein extract. The crude protein extract was washed three times with water: 200 mL of pure water was added to suspend, stirred, left standing, and filtered. After repeated three times, the precipitate was freeze-dried to obtain the target protein CF7.

The same structure confirmation method as in Example 4 confirmed that it had the same amino acid sequence as the protein prepared in Example 4.

### Example 6 : Preparation of protein CF7 by purification from protein crude solution B

The protein crude solution B obtained in Example 2 was purified by the following two methods:

### The first method: dialysis;

The protein crude solution B was filtered with a 0.45 µm membrane, the filtrate was dialyzed with water for more than 72 hours, and the inner solution was freeze-dried to obtain the target protein CF7.

| | |
|---|---|
| Dialysis bag | Molecular weight cutoff: 0.5kD, 3.5kD, 5kD, 10kD |

### The second method: salting out;

The crude protein solution B was placed in a stirred container for salting out twice. Saturated ammonium sulfate solution was slowly added along the wall to make the final concentration of ammonium sulfate reach 25% or 50%. During the salting-out process, the protein was separated out. After the salting-out was completed, the solution was filtered, and the first salting out was finished. 400 mL of pure water was added into the precipitate to suspend, and then a saturated solution of ammonium sulfate was slowly added along the wall again to make the final concentration of ammonium sulfate reach 25%. The second salting out and filtration were carried out, and the precipitate was the crude protein extract. The crude protein extract was washed three times with water. It was suspended in 200 mL of pure water, stirred, allowed to stand, and filtered. After repeated three times, the precipitate was freeze-dried to obtain the target protein CF7.

The product protein CF7 obtained by the two methods was confirmed to have the same amino acid sequence as the protein prepared in Example 4 through the same structural confirmation method as in Example 4.

### Example 7 : Preparation of protein CF7 by purification from protein crude solution C

Crude protein solution C was purified by microfiltration membrane technology: urea was removed by repeated microfiltration using a 20nm or 50nm ceramic membrane core. The inner solution was freeze-dried to obtain the target protein CF7.

The product protein CF7 was confirmed to have the same amino acid sequence as the protein prepared in Example 4 through the same structural confirmation method as in Example 4.

### Pharmacological test

### Experimental example 1 : Pharmacodynamic test of protein CF7 (the protein obtained in Example 7) on yeast-induced SD rat fever model

Animals: Male SD rats, weight: 230-260 grams;
Drugs: yeast (OXOID LP0021), aspirin (SIGMAA2093), protein CF7;
Instruments: electronic balance (SARTORIUS BP121S), electronic thermometer (CITIZEN CT-513W).

### Experiment grouping:

Normal control group;
Model group: yeast fever model group;
Positive control group: Aspirin 300 mg/kg group;
Protein CF7, 10 mg/kg group, 50 mg/kg group.

### Method:

Preparation of experimental animals: after the experimental animals were adapted to the experimental environment (temperature 22°C±2°C, relative humidity 50%±2%) for 1 day, the rectal temperature was measured for pre-adaptation at 8:00 in the morning and 15:00 respectively. The animals were fasted for 12 hours before the experiment with water provided ad libitum, and their bowels were emptied before the rectal temperature was measured. Before each temperature measurement, the probe of the electronic thermometer was applied with Vaseline and inserted 2cm into the rat's rectum (a marker was made at 2 cm to ensure consistent insertion depth each time). The body temperature was recorded after readings stabilized.

Subcutaneous injection of dry yeast to establish a fever model in rats: the body temperature of rats was measured before modeling, and qualified rats with a body temperature of 36.2-37.3°C were screened and randomly divided into groups of 8 rats in each group. After oral administration of aspirin and different doses of protein CF7, 20% yeast suspension (10 mL/kg) was immediately injected subcutaneously. The normal control group was subcutaneously injected with an equal volume of normal saline. The body temperature of rats was monitored 2 hours later, once every 2 hours, for a total of 8 hours.

### Data Statistics :

Based on the body temperature values measured at each time point on the day of the experiment, the mean, standard deviation and standard error of body temperature of rats in each group were calculated. The TTEST test was applied to compare the data of each group between groups. P<0.05 was considered to be significantly different.

### Experimental results:

After oral administration of aspirin (300 mg/kg) and protein CF7 (10 mg/kg, 50 mg/kg), 20% yeast was immediately subcutaneously injected to establish the model. And the body temperature of the animals was monitored at 2 hours, 4 hours, 6 hours, and 8 hours after the establishment of the model, respectively. The results are shown in Table 1 and Figure 1.

**Table 1. Effects of the tested drugs on the yeast-induced fever model in rats**

| Group | N | Basal body temperature (°C) | Body temperature 2 hours after modeling (°C) | Body temperature 4 hours after modeling (°C) | Body temperature 6 hours after modeling (°C) | Body temperature 8 hours after modeling (°C) |
|---|---|---|---|---|---|---|
| Normal control group | 8 | 36.8±0.1 | 36.7±0.1 | 36.8±0.04 | 36.8±0.04 | 36.9±0.1 |
| Model group | 8 | 36.8±0.1 | 37.2±0.2 | 37.6±0.1** | 37.5±0.1 ** | 37.6±0.1** |
| Positive control group | 8 | 36.9±0.1 | 36.8±0.1 | 37.0±0.1# | 37.1±0.1## | 37.1±0.1## |
| CF7 10mg/kg | 8 | 36.8±0.1 | 37.1±0.1 | 37.4±0.1 | 37.2±0.1# | 37.4±0.1 |
| CF7 50mg/kg | 8 | 36.9±0.1 | 36.8±0.1 | 37.4±0.1 | 37.5±0.1 | 37.5±0.1 |

| | | | | | | |
|---|---|---|---|---|---|---|
| (Compared with the normal control group, ** P<0.01; compared with the model group, # P<0.05, ## P<0.01) | | | | | | |

### Experimental conclusions:

After oral administration of aspirin (300 mg/kg) and protein CF7 (10 mg/kg, 50 mg/kg), 20% yeast was immediately subcutaneously injected to establish the model. The body temperature of the animals was monitored at 2 hours, 4 hours, 6 hours, and 8 hours after the establishment of the model, respectively.

The results show:
1) The body temperature of the rats in the model group increased significantly at 2 hours, 4 hours, 6 hours, and 8 hours after modeling, compared with the normal group, P<0.05, with statistically significant differences. The model was successfully established and was stable and reliable.
2) The positive control group drug aspirin can effectively inhibit the increase in body temperature of the model rats at 2 hours, 4 hours, 6 hours, and 8 hours after modeling, compared with the model group, P<0.05, with a statistically significant difference, and the positive control drug aspirin performed stably.
3) The 10mg/kg dose group of protein CF7 can significantly inhibit the temperature elevation in model rats at 6 hours after modeling, and compared with the model group, P<0.05, with a statistically significant difference.

### Experimental example 2 : Pharmacodynamic test of protein CF7 (the protein obtained in Example 7) of antitussive effects on ammonia water-induced cough in mice.

Animals: male ICR mice;
Drugs and reagents: dextromethorphan hydrobromide, ammonia water, 0.2% CMC-Na, protein CF7;
Instruments: Compressed nebulizer (403T), balance (XS105DU).

### Experiment grouping:

Solvent control group;
Dextromethorphan 15 mg/kg group;
Protein CF7, 20 mg/kg group, 50 mg/kg group.

### Method:

### Model establishment and drug administration:

Mice were orally administered dextromethorphan or varying doses of protein CF7 (administration volume: 10 mL/kg). The solvent control group was administered an equal volume of distilled water. After 1 hour, mice were placed in a sealed chamber and atomized with 10% ammonia water for 10 seconds. The cough latency period and cough frequency within 2 minutes were then observed and recorded.

### Data processing:

The time points of oral administration, time points of atomization experiment, the cough latency of mice and the number of coughs within 2 minutes were recorded respectively. The cough latency refers to the number of seconds required from the start of nebulized ammonia solution to the occurrence of cough. The coughing of mice is characterized by the contraction of their abdominal muscles (chest shrinkage) and the opening of their mouths. The mean and standard error of each group of data were calculated, and TTEST was used to compare the model group with other groups. P<0.05 was considered to be significantly different.

### Experimental results:

Pretreatment with dextromethorphan (15 mg/kg) or protein CF7 (20 mg/kg, 50 mg/kg) was administered 1 hour before placing mice in a sealed chamber for 10-second exposure to nebulized 10% ammonia solution. The cough latency and cough frequency within 2 minutes were observed and recorded. Results are shown in Table 2.

**Table 2. Antitussive effect experiment of tested drugs on ammonia water-induced cough in mice (X±SEM)**

| Group | N | Latency (s) | P | Number of coughs | P |
|---|---|---|---|---|---|
| Solvent control group | 9 | 32.0±1.6 | | 61.3±2.5 | |
| Dextromethorphan 15 mg/kg | 9 | 48.4±3.2** | 0.01 | 42.0±2.2** | 0.01 |
| CF7 20mg/kg | 9 | 33.8±3.8 | 0.68 | 56.3±3.9 | 0.30 |
| CF7 50mg/kg | 9 | 33.8±2.3 | 0.54 | 51.5±3.0* | 0.02 |

| | | | | | |
|---|---|---|---|---|---|
| (Compared with the solvent control group, * P<0.05, ** P<0.01) | | | | | |

### Experimental conclusions:

1) The experimental results showed that the dextromethorphan group has a significant improvement in the latency and the number of coughs compared with the solvent control group, P<0.05, with statistically significant.
2) The 50mg/kg dose group of protein CF7 has a statistically significant difference in the number of coughs compared with the solvent control group.

### Experimental example 3 : Pharmacodynamic test of protein CF7 (the protein obtained in Example 7) of expectorant effects by phenol red excretion method in mice

Animals: male ICR mice;
Drugs and reagents: Mucosolvan (ambroxol hydrochloride tablets), phenol red, sodium bicarbonate, protein CF7;
Instruments: centrifuge (Sigma-3K15), balance (XS105DU), Microplate reader (BIO-TEK).

### Experiment grouping:

Solvent control group;
Mucosolvan 30 mg/kg group;
Protein CF7, 20 mg/kg group, 50 mg/kg group.

### Methods:

### Model preparation and drug administration:

Animals were fasted for 16 hours (with free access to water) prior to the experiment. Mucosolvan and different doses of protein CF7 (administered in a volume of 10 mL/kg) were orally administered according to the grouping. The solvent control group was given an equal volume of distilled water. One hour later, 2.5% phenol red solution was intraperitoneally injected. After 30 minutes, the mice were killed by cervical dislocation. A trachea from the below of thyroid cartilage to the front of the tracheal branch was taken, and the trachea was placed in 3 mL of 5% NaHCO₃ solution and allowed to stand for 3 hours. 1 mL of the supernatant was taken, and after centrifugation at 3000 rpm for 5 minutes, the absorbance was measured and recorded at 546 nm. The excretion of phenol red was calculated according to the standard curve of phenol red.

### Data processing:

The time points of oral administration, intraperitoneal injection of the 2.5% phenol red solution, and trachea collection were recorded separately. The absorbance of samples from each group was measured at 546 nm using a microplate reader, and the excretion amount of phenol red was calculated based on its standard curve. The mean and standard error were calculated for each group. Intergroup comparisons between the solvent control group and other groups were performed using the TTEST. P < 0.05 was considered to be statistically significant.

### Experimental results:

After administration of Mucosolvan (30 mg/kg) and different doses of protein CF7 (20 mg/kg, 50 mg/kg), a 2.5% phenol red solution was injected intraperitoneally one hour later. After 30 minutes, the mice were killed by cervical dislocation. A trachea from the below of thyroid cartilage to the front of the tracheal branch was taken, and the trachea was placed in 3 mL of 5% NaHCO₃ solution and allowed to stand for 3 hours. 1 mL of the supernatant was taken, and after centrifugation at 3000 rpm for 5 minutes, the absorbance was measured and recorded at 546 nm. The excretion of phenol red was calculated according to the standard curve of phenol red. The results are shown in Table 3.

**Table 3. Expectorant efficacy experiment of tested drugs by phenol red excretion method in mice (X±SEM)**

| Group | N | Phenol red excretion (µg/mL) | P |
|---|---|---|---|
| Solvent control group | 9 | 0.463±0.036 | |
| Mucosolvan 30 mg/kg | 9 | 0.884±0.061** | 0.01 |
| CF7 20 mg/kg | 9 | 0.576±0.048 | 0.08 |
| CF7 50 mg/kg | 9 | 0.866±0.105** | 0.01 |

| | | | |
|---|---|---|---|
| (Compared with the solvent control group, * P<0.05, ** P<0.01) | | | |

### Experimental conclusions:

1) The experimental results show that compared with the solvent control group, the amount of phenol red excretion in the Mucosolvan 30 mg/kg group is significantly increased, P<0.05, which was statistically significant.
2)Compared to the solvent control group, the excretion of phenol red in the CF7 50 mg/kg group is significantly increased, P<0.01, which was statistically significant.

### Experimental example 4 : Pharmacodynamic test of protein CF7 (the protein obtained in Example 7) on acetic acid-induced writhing in ICR mice

Animals: male ICR mice;
Drugs and reagents: aspirin, physiological saline, glacial acetic acid, protein CF7.

### Experiment grouping:

Model group;
Aspirin 300 mg/kg group;
Protein CF7, 50 mg/kg group, 200 mg/kg group.

### Methods:

After one day of acclimation, the experimental animals were orally administered aspirin 300 mg/kg, protein CF7 50 mg/kg or protein CF7 200 mg/kg one hour in advance (administration volume: 10 mL/kg). Then 0.6% acetic acid solution was injected intraperitoneally, and the latency (seconds) and number of writhings in the animals were observed within 15 minutes.

### Data Processing:

The mean and standard error was calculated for the data of each group. Intergroup comparisons with the model group were performed using TTEST. P<0.05 was considered statistically significant.

### Experimental Results:

One hour after oral administration of aspirin 300 mg/kg or different doses of protein CF7 (50 mg/kg, 200 mg/kg), 0.6% acetic acid solution was injected intraperitoneally. The latency and the number of writhings in ICR mice were observed. The results are shown in Table 4.

**Table 4. Effects of tested drug on the acetic acid-induced writhing test in ICR mice.**

| Group | N | Weight (g) | Writhing latency (seconds) | Number of writhings (times) |
|---|---|---|---|---|
| Model group 0.6% acetic acid | 15 | 25.3±0.1 | 193.5±15.0 | 34.6±3.1 |
| aspirin 300 mg/kg | 12 | 25.3±0.2 | 293.7±26.7** | 18.1±2.9** |
| CF7 50 mg/kg | 10 | 24.9±0.2 | 284.4±85.4 | 28.5±7.1 |
| CF7 200 mg/kg | 11 | 25.2±0.2 | 177.0±11.1 | 29.8±5.7 |

| | | | | |
|---|---|---|---|---|
| (Compared with the model group, * P<0.05, ** P<0.01) | | | | |

### Experimental conclusions:

0.6% acetic acid solution was injected into the abdominal cavity of mice, causing deep, large-area and long-lasting pain stimulation, which caused the mice to have a writhing reaction (the abdomen contracted into an "S" shape, the trunk and hind legs stretched, the buttocks raised and crawled). The latency and the number of times the mice began to writhe were used as pain reaction indicators to determine whether the test sample had analgesic effect. The experimental results show that:
1) Aspirin 300 mg/kg can significantly delay the writhing latency and reduce the number of writhings, and has a certain analgesic effect. Compared with the model group, P<0.05, which is statistically significant.
2) The sample protein CF7 50 mg/kg shows a certain trend of delaying the animal's writhing latency and reducing the number of writhing times, but there is no statistical difference compared with the model group.

## Claims

1. A keratin CF7, wherein the amino acid sequence of the keratin CF7 is:
(1) the amino acid sequence shown in SEQ ID NO. 1 in the sequence listing; or
(2) an amino acid sequence obtained by substituting, deleting, or adding 1 to 45 amino acids in the amino acid sequence shown in SEQ ID NO. 1 in the sequence listing, and the keratin CF7 substantially maintains the same biological function.

2. The keratin CF7 according to claim 1, wherein conventional modifications can be made to the keratin CF7; or a tag for detection or purification is also attached to the keratin CF7; or is a homologous protein of the keratin CF7.

3. The keratin CF7 according to claim 2, wherein the conventional modification is acetylation, amidation, cyclization, glycosylation, phosphorylation, alkylation, biotinylation, fluorophore modification, polyethylene glycol PEG modification, immobilization modification, sulfation, oxidation, methylation, deamidation, disulfide bond formation, or disulfide bond cleavage; or the tag is His6, GST, EGFP, MBP, Nus, HA, IgG, FLAG, c-Myc, or Profinity eXact.

4. A nucleic acid molecule encoding the keratin CF7 according to any one of claims 1 to 3.

5. The nucleic acid molecule according to claim 4, wherein the nucleotide sequence of the nucleic acid molecule is:
(1) the nucleotide sequence shown in SEQ ID NO. 2 in the sequence listing;
(2) a nucleotide sequence obtained by sequence optimization based on the nucleotide sequence shown in SEQ ID NO. 2; or
(3) a nucleotide sequence complementary to the nucleotide sequence in (1) or (2) above.

6. An expression vector, wherein the expression vector comprises the nucleic acid molecule according to any one of claims 4 to 5.

7. A host cell, wherein the host cell comprises the expression vector according to claim 6, or the nucleic acid molecule according to any one of claims 4-5 is integrated into the genome of the host cell.

8. The host cell according to claim 7, wherein the host cell is a bacteria, a yeast, an aspergillus, a plant cell, or an insect cell.

9. The host cell according to claim 8, wherein the bacteria is *Escherichia coli.*

10. A method for preparing the keratin CF7 according to any one of claims 1 to 3, comprising the following steps:
A. the nucleic acid molecule corresponding to the keratin CF7 according to any one of claims 1 to 3 is synthesized, the nucleic acid molecule is ligated into a corresponding expression vector, the expression vector is transformed into a host cell, the host cell carrying the expression vector is cultured under a certain condition in a fermentation device, and the expression of keratin CF7 is induced, to obtain a crude protein solution containing keratin CF7; and
B. the crude protein solution expressed in step A is separated, purified, and dried to obtain keratin CF7.

11. The method according to claim 10, wherein, in step A, the host cell is primarily *Escherichia coli,* the keratin CF7 is expressed in Escherichia coli inclusion bodies, and the fermentation device is a shake flask or a fermenter.

12. The method according to claim 10, wherein, in step A, after inducing the expression of keratin CF7, impurities can be washed with a cleaning agent, dissolved with a solution to obtain the crude protein solution.

13. The method according to claim 10, wherein, in step B, the separation and purification method is ultrafiltration and microfiltration membrane technology purification method, column chromatography purification method, salting-out method, or dialysis method.

14. A pharmaceutical composition, wherein the pharmaceutical composition comprises the keratin CF7 according to any one of claims 1 to 3 and a pharmaceutically acceptable carrier or excipient.

15. Use of the keratin CF7 according to any one of claims 1 to 3, or the nucleic acid molecule according to any one of claims 4 to 5, or the expression vector according to claim 6, or the host cell according to any one of claims 7 to 9, or the pharmaceutical composition according to claim 14 in the preparation of a medicament for antipyretic, analgesic, antitussive, expectorant, anticonvulsant, antiepileptic, hypotensive, anti-inflammatory, or antiviral therapy.
